# EUROPÄISCHE PATENTSCHRIFT

(11) **EP 3 613 347 B1**
(45) Veröffentlichungstag und Bekanntmachung des Hinweises auf die Patenterteilung: **17.03.2021**
(21) Anmeldenummer: 18190507.6
(22) Anmeldetag: 23.08.2018
(51) Int. Cl.: A61B 6/04, A61G 13/12

(54) **PATIENTENLAGERUNGSEINRICHTUNG FÜR EINE RÖNTGENEINRICHTUNG UND VERFAHREN ZUR ANPASSUNG EINER PATIENTENLIEGE EINER PATIENTENLAGERUNGSEINRICHTUNG**
PATIENT SUPPORTING DEVICE FOR AN X-RAY DEVICE AND METHOD FOR ADAPTATION OF A PATIENT'S COUCH OF A PATIENT SUPPORTING DEVICE
DISPOSITIF DE POSITIONNEMENT DU PATIENT POUR UN DISPOSITIF À RAYONS X ET PROCÉDÉ D'AJUSTEMENT D'UNE COUCHETTE DU PATIENT D'UN DISPOSITIF DE POSITIONNEMENT DU PATIENT

(43) Veröffentlichungstag der Anmeldung: 26.02.2020
(73) Patentinhaber: Siemens Healthcare GmbH, 91052 Erlangen (DE)
(72) Erfinder: Wiets, Michael, 91094 Langensendelbach (DE)

(56) Entgegenhaltungen:
- DE-A1- 3 716 263
- DE-A1- 19 900 257
- US-A1- 2017 135 884

## Beschreibung

Die Erfindung betrifft eine Patientenlagerungseinrichtung für eine Röntgeneinrichtung zum Einsatz bei einer Untersuchung eines Patienten, aufweisend eine Patientenliege und wenigstens einen die Patientenliege tragenden Träger. Daneben betrifft die Erfindung ein Verfahren zur Anpassung der Patientenliege einer solchen Patientenlagerungseinrichtung.

Bei der Durchführung von Untersuchungen, insbesondere im Rahmen von chirurgischen Eingriffen, also Operationen, oder von Therapiemaßnahmen, wurden bereits eine Vielzahl von Patientenlagerungseinrichtungen vorgeschlagen, um den Patienten zum einen sicher und stabil, zum anderen so zu lagern, dass eine für die Untersuchung, insbesondere den chirurgischen Eingriff, optimale Positionierung des Patienten vorliegt. Hierzu weisen Patientenlagerungseinrichtungen dieser Art, die auch als Operationstische bezeichnet werden können, meist eine Vielzahl von Verstelleinrichtungen auf, insbesondere Gelenkverbindungen zwischen unterschiedlichen Platten der Patientenliege, die die Lagerungsfläche für den Patienten bereitstellt.

Zunehmend werden für chirurgische Eingriffe, insbesondere für minimalinvasive Eingriffe, also Operationen eines Patienten, Bildüberwachungsmechanismen vorgeschlagen, die meist über Röntgeneinrichtungen bereitgestellt werden sollen. In diesem Fall ist die Untersuchung des Patienten also operationsbegleitend. Beispielsweise wurde vorgeschlagen, die Position eines minimalinvasiven medizinischen Instruments innerhalb eines Patienten durch Fluoroskopiebilder einer Röntgeneinrichtung nachzuverfolgen. Dabei kann beispielsweise eine Röntgeneinrichtung mit einem C-Bogen verwendet werden, die in einer Vielzahl von Aufnahmegeometrien einstellbar ist. Problematisch in diesem Zusammenhang ist bei einer Verwendung der beschriebenen, viele Verstellmöglichkeiten aufweisenden Operationstische als Patientenlagerungseinrichtung für die Röntgeneinrichtung, dass diese Operationstische meist nicht oder nur teilweise röntgendurchlässig sind. Beispielsweise können Gelenke, die einzelne Platten der Patientenliege miteinander verbinden, Edelstahl aufweisen, womit die Gelenke nicht röntgentransparent sind. Dabei zeigt es sich in wissenschaftlichen Untersuchungen, dass durch die Patientenliegen der Operationstische, insbesondere im Bereich von Verstelleinrichtungen, die Dynamik der Röntgenbilder derart verschlechtert wird, dass sich kein nutzbringendes Röntgenbild der Anatomie des Patienten mehr aufnehmen lässt.

Um dieses Problem zu lösen, wurden röntgentransparente Patientenliegen (Tischplatten) vorgeschlagen, die dann aber konsequenterweise keine bzw. nur sehr wenige Verstellmöglichkeiten aufweisen. Dies kann die Operationen an dem Patienten jedoch deutlich erschweren, was unerwünscht ist. Idealerweise verfügt nämlich ein mehrfach verstellbarer Operationstisch über Gelenke an den jeweils großen Gelenken eines Patienten, insbesondere Gelenke entsprechend Kniegelenken, Hüftgelenken und Schultergelenken. Teilweise werden Erweiterungen wie Armhalter und/oder Kopfhalter angebracht. Optimal wäre eine längs teilbare Patientenliege im Beinbereich, um beispielsweise laparoskopische Eingriffe besser durchführen zu können.

Der Erfindung liegt daher die Aufgabe zu Grunde, eine Patientenlagerungseinrichtung für Röntgen-Untersuchungen, insbesondere die Röntgen-Bildüberwachung bei Operationen, an einem Patienten anzugeben, die trotz möglichst flexibler Verstellbarkeit weiterhin die Aufnahme hochqualitativer Röntgenbilder erlaubt.

Zur Lösung dieser Aufgabe ist bei einer Patientenlagerungseinrichtung der eingangs genannten Art erfindungsgemäß vorgesehen, dass die Patientenliege ein erstes, röntgentransparentes, in einer wenigstens teilweise flexiblen Hülle aus einem zweiten, röntgentransparenten Material befindliches Material aufweist, wobei das erste Material einen festen, formstabilen Zustand und einen formbaren Zustand, insbesondere wenigstens teilweise flüssigen und/oder gasförmigen Zustand, aufweist, die durch reversible Phasenübergänge verknüpft sind, wobei die Patientenlagerungseinrichtung ferner wenigstens eine Induzierungseinrichtung zur Induzierung wenigstens eines der Phasenübergange des ersten Materials zwischen den Zuständen durch einen Energieeintrag und/oder Energieentzug aufweist.

Dabei ist im Rahmen der vorliegenden Erfindung der Begriff "röntgentransparent" breit zu verstehen und bedeutet im Wesentlichen, dass ein für eine bildgebende Messung ausreichender Anteil der Röntgenstrahlung das erste und das zweite Material durchquert. Beispielsweise können das erste und/oder das zweite Material eine mit Fett und/oder Wasser vergleichbare Röntgenschwächung aufweisen, so dass dennoch eine hinreichende Röntgentransparenz gegeben ist, insbesondere bei anvisierten Dicken der Patientenliege im Bereich von wenigen Zentimetern, beispielweise im Bereich von 2 bis 7 cm. Dabei ist die Dicke der Patientenliege zumindest im Wesentlichen überall gleich, so dass die Schwächung durch die Materialien an jeder Stelle zumindest im Wesentlichen gleich groß ist.

Das erste Material weist dabei einen festen, formstabilen und einen formbaren Zustand auf, die durch reversible Phasenübergängen verknüpft sind, das heißt, wenn das erste Material in einem der beiden Zustände vorliegt, kann es durch einen Phasenübergang in den anderen Zustand überführt werden. Nachdem dies reversibel ist, gilt es auch umgekehrt.

Die Verwendung eines solchen Materials, das durch einen Phasenübergang in einen anderen Zustand überführt werden kann, für eine Patientenlagerungseinrichtung ist grundsätzlich bereits aus den Schriften US 2017/135884 A1, DE 199 00 257 A1 und DE 37 16 263 A1 bekannt.

Erfindungsgemäß wird also vorgeschlagen, die lösbar mit dem Träger, insbesondere wenigstens einer Säule, verbundene Patientenliege, die auch als Patientenplatte bezeichnet werden kann, nicht wie bisher aus Teilplatten, die die Last des Patienten aufnehmen, und Gelenken, die die einzelnen Teilplatten miteinander verbinden, auszubilden, sondern aus einer durchgängigen Hülle aus einem zweiten Material mit einem ersten Material darin, das, beispielsweise in einem geeigneten Temperaturbereich, einen von außen induzierbaren Phasenübergang, beispielsweise von flüssig zu fest, durchläuft. Dabei ist zweckmäßigerweise das zweite Material im für das erste Material durch die Induzierungseinrichtung genutzten Zustandsbereich phasenstabil. Dabei sei bereits an dieser Stelle darauf hingewiesen, dass zumindest einer der Phasenübergänge auch ohne Induzierung stattfinden kann, worauf im Folgenden noch näher eingegangen werden wird. Die grundlegende Idee ist es also, bei einem formbaren Zustand des ersten Materials dank der flexiblen Hülle die Patientenliege zunächst in einer gewünschten Art zu formen, um sie anschließend, insbesondere induziert, durch einen Phasenübergang in den festen, formstabilen Zustand überzuführen, sie mithin in der vorgegebenen Form erstarren zu lassen. Anschließend kann der Patient auf der Patientenliege, die gegebenenfalls an dem Träger zu befestigen ist, der Untersuchung, insbesondere gemeinsam mit bzw. begleitend zu einem chirurgischen Eingriff, also einer Operation, unterzogen werden. Für den nächsten Patienten (oder eine andere Untersuchung) kann dann mittels eines weiteren Phasenübergangs das erste Material wieder von dem festen in den formbaren Zustand übergeführt werden, so dass eine erneute Formanpassung erfolgen kann.

Auf diese Weise wird eine Patientenlagerungseinrichtung, die auch als Patiententisch bezeichnet werden kann, für die Durchführung einer Untersuchung, insbesondere gemeinsam mit bzw. begleitend zu einem chirurgischen Eingriff, an einem Patienten bei Röntgenüberwachung bereitgestellt, die flexibel auf unterschiedliche ideale Formen für die Untersuchung und/oder Behandlung des Patienten einstellbar ist, dennoch jedoch eine Röntgentransparenz aufweist, die eine hervorragende Röntgenbildgebung auch durch die Patientenliege hindurch erlaubt.

Dabei ist, wie bereits dargelegt wurde, die Patientenliege bevorzugt lösbar an dem Träger befestigbar, das bedeutet, die Patientenlagerungseinrichtung weist eine lösbare Befestigungseinrichtung zur Befestigung der Patientenliege an dem Träger auf. Die lösbare Befestigungseinrichtung kann bevorzugt ebenfalls wenigstens teilweise röntgentransparent sein und/oder eine möglichst geringe Befestigungsfläche nutzen, insbesondere weniger als 10 % der zur Lagerung des Patienten bereitstehenden Oberfläche der Patientenliege, bevorzugt weniger als 5 % der zur Lagerung des Patienten bereitstehenden Oberfläche der Patientenliege. Der Träger weist bevorzugt eine oder mehrere Säulen auf und/oder kann die Patientenliege frei tragen. Bei einer Säule befindet sich diese bevorzugt mittig unterhalb bzw. im Schwerpunkt der Patientenliege, so dass sich auf die patientenliegenseitigen Befestigungsmittel der lösbaren Befestigungseinrichtung bevorzugt mittig bzw. im Schwerpunkt an der der zur Lagerung des Patienten bereitstehenden Lagerungsfläche gegenüberliegenden Oberfläche befindet. Bei mehreren Säulen kann beispielsweise vorgesehen sein, jeweils wenigstens eine Säule an jeweils einem Längsende der Patientenliege vorzusehen.

Die Patientenliege kann konkret in die durchgängige Hülle, also das zweite Material, integrierte Befestigungsmittel der lösbaren Befestigungseinrichtung aufweisen, beispielsweise eine in das Trägermaterial eingelassene, beispielsweise eingegossene und/oder an dem Trägermaterial befestigte, beispielsweise verklebte, Platte, die Muttern für in diese zur Befestigung eingreifende Schrauben der lösbaren Befestigungseinrichtung bietet. Ein anderes Beispiel für die Befestigungsmittel nutzt Vorsprünge/Profile, die in entsprechend ausgeformte Nuten einschiebbar sind. Selbstverständlich sind auch andere entsprechende Ausgestaltungen denkbar.

In bevorzugter Weiterbildung der vorliegenden Erfindung kann vorgesehen sein, dass das erste Material im formbaren Zustand zähflüssig ist. Beispielsweise kann das erste Material eine Viskosität größer als 100 mPas aufweisen. Eine derartige Zähflüssigkeit bzw. hohe Viskosität trägt im Übrigen auch dazu bei, dass es nicht zu großen Umverteilungen des ersten Materials kommt, dieses mithin über die Fläche der Patientenliege eine zumindest im Wesentlichen gleiche Ausdehnung während der Verformung beibehält. Weitere zu dieser vorteilhaften Ausgestaltung beitragende Merkmale der Patientenliege werden im Folgenden noch näher diskutiert werden.

In einer ersten, beispielhaften Ausgestaltungsform kann das erste Material Wasser sein. Bei normalen Raumtemperaturen ist Wasser flüssig, wobei es beim Abkühlen zu Eis erstarrt. Dieses Abkühlen kann beispielsweise durch die Induzierungseinrichtung herbeigeführt werden. Dabei kann vorgesehen sein, die Hülle wärmeisolierend zu gestalten, wobei eine leichte Unterkühlung bei vielen Arten von Operationen auch durchaus vorteilhaft sein kann.

In einer gegenüber der Verwendung von Wasser bevorzugten Ausgestaltung kann das erste Material ein Latentwärmespeichermaterial, insbesondere Natriumacetat-Trihydrat und/oder ein sonstiges Paraffin und/oder ein PCM-Wachs, umfassen oder sein. Latentwärmespeichermaterialien sind auch als Phasenwechselmaterialien (englisch PCM - Phase Change Materials) bekannt. Sie werden heute beispielsweise in sogenannten Handwärmern eingesetzt und weisen bevorzugt einen Phasenübergang zwischen einem zähflüssigen und einem kristallisierten, festen Zustand auf. Bei Latentwärmespeichermaterialien wie beispielsweise Paraffin, insbesondere Natriumacetat-Trihydrat, und/oder PCM-Wachs, handelt es sich um Phasenwechselmaterialien, bei denen sich die Induzierung eines Phasenübergangs, insbesondere in wenigstens einer Richtung, besonders einfach erreichen lässt. Im Beispiel des Natriumacetats-Trihydrats weist dieses eine Schmelztemperatur von 58°C auf, so dass durch Erwärmen der Patientenliege auf diese Temperatur der zähflüssige Zustand herbeigeführt werden kann, welcher auch noch bei Temperaturen weit unterhalb des Schmelzpunktes, unter Umständen bis -20°C, als unterkühlte Schmelze in einem metastabilen Zustand erhalten bleibt, da sich das Salz in seinem Kristallwasser löst. Wird nun als Induzierungseinrichtung eine Kristallisationsauslösevorrichtung, beispielsweise ein Knackplättchen und/oder ein Piezoelement, verwendet, kann die Kristallisation ausgelöst werden, wobei sich allerdings die Patientenliege dann wieder erwärmt und zweckmäßigerweise die Patientenlagerungseinrichtung eine Wärmeabführungseinrichtung/Kühleinrichtung aufweist, um die bei der Erstarrung entstehende Wärme abzuführen. Zudem ist auch in diesem Ausführungsbeispiel ein elastisch dehnbares zweites Material der Hülle zweckmäßig, nachdem beispielsweise Paraffine eine Volumenänderung von etwa 10 bis 30 % beim Phasenübergang von fest nach flüssig aufweisen, welche bei der Wahl des zweiten Materials der Hülle berücksichtigt werden muss.

Ein anderes zweckmäßiges Latentwärmespeichermaterial neben dem erwähnten Natriumacetat-Trihydrat, das im Rahmen der Erfindung besonders geeignet ist, ist sogenannter PCM-Wachs, beispielsweise das unter dem Namen RT25 von der Firma Rubitherm Technologies GmbH, Berlin, Deutschland vertriebene Material. Dieses weist einen geeigneten Schmelzpunkt von etwa 22-26° C auf.

In einem weiteren Beispiel wird in einer bevorzugten Ausgestaltung ein Schaum als erstes Material bzw. ein einen Schaum umfassendes erstes Material verwendet. Schaum bietet sich insbesondere als Trägermaterial an, in das ein Latentwärmespeichermaterial eingebettet sein kann, was auch Gegenstand laufender Forschungen ist. Schäume können die Wärmespeicher- und Wärmeabgabeeigenschaften regulierend beeinflussen und somit gerade im Rahmen der vorliegenden Erfindung, in der die Wärmespeicherungseigenschaft weniger wesentlich ist, nutzbringend eingesetzt werden.

Ein anderes Beispiel sieht vor, dass das erste Material eine Flüssigkeit ist oder umfasst, die unter dem Einfluss eines elektrischen und/oder magnetischen Feldes ihre Viskosität ändert. Solche Flüssigkeiten werden auch als elektrorheologische bzw. magnetorheologische Flüssigkeiten bezeichnet. In diesem Fall kann die Induzierungseinrichtung beispielsweise einen entsprechenden Feldgenerator umfassen.

Als das zweite Material kann im Rahmen der vorliegenden Erfindung wenigstens ein Kunststoff verwendet werden und/oder das zweite Material kann wenigstens einen Kunststoff umfassen. Geeignete Kunststoffe, die die nötige Flexibilität sowie gegebenenfalls Elastizität aufweisen sowie im relevanten Einsatzbereich phasenstabil sind, sind im Stand der Technik in großer Zahl bereits bekannt und müssen hier nicht näher dargelegt werden.

In einer zweckmäßigen Weiterbildung der vorliegenden Erfindung kann vorgesehen sein, dass das erste Material und das zweite Material im Wesentlichen gleiche Schwächungseigenschaften für Röntgenstrahlung aufweisen. Auf diese Weise werden Übergänge unterschiedlicher Schwächungseigenschaften, die zur Brechung und/oder zu anderen Effekten führen können, möglichst weitgehend vermieden und der ohnehin auf Grund der Röntgentransparenz geringe Einfluss der Patientenliege wird weiter reduziert. Artefakte in der Bildgebung werden minimiert.

In besonders bevorzugter Weiterbildung der vorliegenden Erfindung kann vorgesehen sein, dass das zweite Material zur Kompensation einer bei einem der Phasenübergänge auftretenden Volumenänderung und/oder zur Begünstigung einer Gleichverteilung einer Materialstärke des ersten Materials elastisch ausgebildet ist und/oder wenigstens einen zur Formstabilisierung ausgebildeten, Durchtrittsöffnungen für das erste Material aufweisenden Steg aufweist und/oder nur an Sollgelenkstellen flexibel ist. Der Begriff Steg ist hierbei breit zu verstehen. So können entsprechend durch Stege begrenzte Waben entstehen, die beispielsweise eine Größe im Bereich von 20 bis 40 mm, beispielsweise 30 mm, haben können. Die Waben können hexagonal ausgebildet sein und insbesondere dort mit Öffnungen versehen sein.

Nachdem einige verwendbare, nützliche erste Materialien bei den Phasenübergängen Volumenänderungen aufweisen können, ist es zweckmäßig, eine gewisse, dies kompensierende Elastizität des ersten Materials vorzusehen. Eine derartige Elastizität ist jedoch auch zweckmäßig, um zu einer bestimmten Dicke der Patientenliege zurückzustreben, mithin deren Gesamtdicke über die Fläche möglichst konstant zu halten.

Besonders zweckmäßig ist es jedoch auch, zusätzlich oder alternativ innerhalb der Hülle wenigstens einen zur Formstabilisierung ausgebildeten, Durchtrittsöffnungen für das erste Material aufweisenden Steg vorzusehen. Derartige, stabilisierende, dennoch einen Austausch des ersten Materials erlaubende Stege sind beispielsweise aus dem Bereich der Luftmatratzen bekannt. In einer beispielhaften Ausgestaltung, gemäß der Formänderungen der Patientenliege hauptsächlich entlang der Längsrichtung der Patientenliege erfolgen sollen, kann beispielsweise vorgesehen sein, Stege in Querrichtung in Bereichen der Patientenliege vorzusehen, in denen ein kurzstreckiger Verlust der Flexibilität wenig relevant ist. Selbstverständlich sind auch, wenn Formänderungen in Querrichtung gewünscht sind, nur einen Teil der Patientenliege in Querrichtung abdeckende Stege denkbar.

In einer anderen Ausgestaltung ist es grundsätzlich auch denkbar, jedoch weniger bevorzugt, wenn das zweite Material nur an Sollgelenkstellen flexibel ist, das bedeutet, Stellen, an denen eine Verformung auftreten kann, von vornherein festgelegt sind. Außerhalb dieser Sollgelenkstellen wird das erste Material unflexibel gewählt, so dass hier auch eine gleichbleibende Dicke der Patientenliege sichergestellt wird. Dies erfordert jedoch eine über die Ausdehnung der Patientenliege unterschiedliche Ausgestaltung des zweiten Materials, was herstellungstechnisch weniger bevorzugt ist, und kann zu einer Reduzierung der gegebenen Flexibilität in der Anpassung der Patientenliege führen.

Die Induzierungseinrichtung kann zweckmäßigerweise, je nach Art des Phasenübergangs, zur Erzeugung des Energieeintrags bzw. Energieentzugs durch Wärme und/oder Kälte und/oder elektrischen Strom ausgebildet sein. Beispielsweise kann die Induzierungseinrichtung mithin als Heizeinrichtung und/oder Kühleinrichtung und/oder Kristallationsauslösevorrichtung, insbesondere Knackplättchen und/oder Piezoelement, und/oder Bestromungseinrichtung, letztere insbesondere aufweisend wenigstens zwei Elektroden, ausgebildet sein. Bei einem zu schmelzenden ersten Material kann beispielsweise eine Heizeinrichtung als Induzierungseinrichtung verwendet werden, um die hinreichende Wärme zuzuführen. Entsprechend kann aber auch mit dem umgekehrten Phasenübergang eine Kühleinrichtung als gegebenenfalls weitere Induzierungseinrichtung vorgesehen werden. Kristallationsauslösevorrichtungen sind, wie bereits dargelegt wurde, insbesondere dann sinnvoll, wenn induziert, beispielsweise unter Wärmeabgabe, kristallisierend erstarrende erste Materialien verwendet werden sollen, beispielsweise Paraffin. Hier bieten sich insbesondere die beispielsweise aus Wärmekissen bekannten Ausbildungen als Knackplättchen und/oder Piezoelement an. Wenn das erste Material zur Erstarrung Wärme abgibt, kann zusätzlich zu der Kristallationsauslösevorrichtung auch eine Kühleinrichtung als Wärmeabführungseinrichtung vorgesehen werden.

Gemäß der vorliegenden Erfindung ist vorgesehen, dass die Patientenlagerungseinrichtung eine Vorbereitungseinrichtung für die Patientenliege aufweist, wobei die Vorbereitungseinrichtung aufweist:
- wenigstens eine Auflagefläche für die Patientenliege und
- wenigstens eine auf die Auflagefläche wirkende Verstelleinrichtung.

Die Auflagefläche ist dabei bevorzugt in ihrer Ausdehnung auf die Ausdehnung der Patientenliege angepasst. Wird die Patientenliege mit dem ersten Material in einem formbaren Zustand auf die Auflagefläche aufgebracht, können die Verstelleinrichtungen der Vorbereitungseinrichtung genutzt werden, um gezielt und definiert Verformungen der Patientenliege herbeizuführen. Dabei können die Verstelleinrichtungen beispielsweise zur Verstellbarkeit an allen für Operationen an dem Patienten, die röntgenbildüberwacht werden sollen, nützlichen Positionen vorgesehen werden.

In konkreter Ausgestaltung kann wenigstens eine der wenigstens eine Verstelleinrichtung ein Gelenk zur Verkippung eines Anteils der Auflagefläche gegen einen benachbarten Anteil der Auflagefläche sein. Selbstverständlich sind jedoch auch andere Ausbildungen der Verstelleinrichtung grundsätzlich möglich.

Die Vorbereitungseinrichtung kann ein Operationstisch oder ein Gestell sein. Ein (wenigstens teilweise nicht röntgentransparenter) Operationstisch bietet den Vorteil, dass die entsprechenden Verstellmöglichkeiten meist ohnehin bereits gegeben sind, wobei insbesondere auch der Operationstisch mit dem eigentlichen Patienten vorbereitet werden kann, wonach letztlich eine Art "Abdruck" der Einstellungen des Operationstisches mit Hilfe der Patientenliege genommen werden kann. Mithin können die Verstellmöglichkeiten des Operationstisches sozusagen auf die Patientenlagerung an der Röntgeneinrichtung übernommen werden, wobei zusätzlich die Röntgentransparenz hergestellt wird.

Alternativ kann, insbesondere auf kostengünstigere Weise, als Vorbereitungseinrichtung auch ein Gestell bereitgestellt werden, welches bei deutlich geringeren Anforderungen an die mechanische Robustheit und Stabilität dennoch die verschiedenen gewünschten Anformmöglichkeiten für die Patientenliege aufweisen kann. Beispielsweise können in dem Gestell typische Verstellmöglichkeiten für Operationstische umgesetzt werden, beispielsweise Verstelleinrichtungen, die in ihrer Lage der von Gelenken des Patienten entsprechen.

Als besonders zweckmäßig hat es sich erwiesen, wenn die Patientenliege in einem zur Lagerung der Beine des Patienten vorgesehenen Beinbereich einen in Längsrichtung verlaufenden Einschnitt zur unabhängigen Einstellung der Lagerfläche für die jeweiligen Beine eines Patienten in dem formbaren Zustand aufweist. Das bedeutet, unterhalb der Lage des Hüftgelenks des Patienten kann die Patientenliege in Querrichtung zweigeteilt sein, mithin ein Einschnitt in Längsrichtung vorgesehen werden, so dass es möglich ist, bei der Formgebung im formbaren Zustand unterschiedliche Einstellungen für die unterschiedlichen Beine des Patienten vorzunehmen, beispielsweise ein Bein nach unten abgewinkelt zu lagern, was bei einer Laparoskopie von besonderem Vorteil sein kann. Ein anderes Beispiel betrifft die aufgespreizte Lagerung der Beine, das bedeutet, wenigstens ein Bein wird nach seitlich abgewinkelt gelagert. Hierzu kann zusätzlich zu dem Einschnitt in Längsrichtung, insbesondere an dessen Ende anschließend, wenigstens ein Einschnitt auch in Querrichtung vorgesehen sein. Zweckmäßigerweise kann, falls vorgesehen, die Vorbereitungseinrichtung entsprechende Verstelleinrichtungen für die jeweiligen Beinanteile der Patientenliege aufweisen.

An dem Träger können im Übrigen ebenso Möglichkeiten zur Einstellung, konkret mithin Einstellungseinrichtungen, vorgesehen werden. So hat es sich als besonders zweckmäßig erwiesen, wenn der insbesondere als wenigstens eine Säule ausgebildete Träger wenigstens in seiner Höhe verstellbar ist. So kann eine weitere Anpassung an die konkreten Operationsumstände erfolgen. Andere denkbare Einstellungseinrichtungen können auch eine Verkippung der Patientenliege ermöglichen.

Es sei noch angemerkt, dass, insbesondere im Hinblick auf Untersuchungen im Rahmen einer Operation und/oder Therapie, die Patientenlagerungseinrichtung, insbesondere die Patientenliege, auch übliche Fixierungsmittel zur Fixierung des Patienten aufweisen kann.

Neben der Patientenlagerungseinrichtung betrifft die vorliegende Erfindung auch ein Verfahren zur Anpassung der Patientenliege einer solchen Patientenlagerungseinrichtung, welches folgende Schritte umfasst:
- Herstellen des formbaren Zustands des zweiten Materials, insbesondere durch Induzierung eines Phasenübergangs
- Lösen der Patientenliege vom Träger und Platzieren der Patientenliege auf der Auflagefläche der Vorbereitungseinrichtung,
- Formanpassung der Patientenliege, mittels einer Verstelleinrichtung,
- Herstellen des festen Zustands des zweiten Materials, insbesondere durch Induzierung eines Phasenübergangs,
- Befestigung der Patientenliege an dem Träger.

Sämtliche Ausführungen bezüglich der erfindungsgemäßen Patientenlagerungseinrichtung lassen sich analog auf das erfindungsgemäße Verfahren übertragen, mit welchem mithin ebenso die bereits genannten Vorteile erhalten werden können.

Insbesondere wird auch beim erfindungsgemäßen Verfahren eine Vorbereitungseinrichtung verwendet, wobei zunächst mittels der lösbaren Befestigungseinrichtung die Patientenliege von dem Träger gelöst wird, die Patientenliege auf der Auflagefläche der Vorbereitungseinrichtung platziert wird und die Formanpassung mit Hilfe der Verstelleinrichtung durchgeführt wird. Nachdem der feste Zustand des zweiten Materials, insbesondere durch Verwendung einer Induzierungseinrichtung, wieder hergestellt wurde, kann mittels der lösbaren Befestigungseinrichtung die Patientenliege wieder an dem Träger befestigt werden. Danach ist es möglich, den Patienten zu positionieren und mit der Operation an dem Patienten zu beginnen, welche durch die Röntgeneinrichtung auf Grund der Röntgentransparenz der Patientenliege in hoher Qualität überwacht werden kann.

Dabei sei an dieser Stelle noch angemerkt, dass zumindest in einigen Ausführungsbeispielen der Phasenübergang nur in einer Richtung induziert werden muss; beispielsweise kann bei Wasser als erstes Material die Patientenliege letztlich nach der Verwendung "automatisch" wieder auftauen und das erste Material in den formbaren Zustand übergehen.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: eine erfindungsgemäße Patientenlagerungseinrichtung mit einer Röntgeneinrichtung,
- Fig. 2: eine Patientenliege der Patientenlagerungseinrichtung in einem Querschnitt,
- Fig. 3: eine Aufsicht auf die Patientenliege,
- Fig. 4: eine Seitenansicht eines Auflagebereichs eines Gestells der Patientenlagerungseinrichtung,
- Fig. 5: eine Aufsicht auf den Auflagebereich,
- Fig. 6: eine Darstellung zur Anpassung der Patientenliege, und
- Fig. 7: die angepasste Patientenliege im Einsatz als Patiententisch.

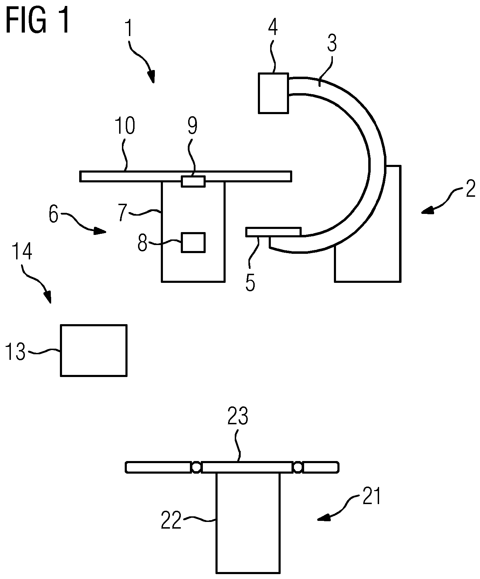

Fig. 1 zeigt eine Prinzipskizze wesentlicher Komponenten einer erfindungsgemäßen Patientenlagerungseinrichtung 1, die zum Einsatz bei einer Operation an einem Patienten, also einem chirurgischen Eingriff, unter Röntgenüberwachung durch eine hier ebenso angedeutete Röntgeneinrichtung 2 verwendet werden soll. Die Röntgeneinrichtung 2 ist dabei beispielhaft als eine Röntgeneinrichtung 2 mit einem C-Bogen 3 gezeigt, an dem sich gegenüberliegend ein Röntgenstrahler 4 und ein Röntgendetektor 5 angeordnet sind.

Die Patientenlagerungseinrichtung 1 weist einen im Bereich der Röntgeneinrichtung 2 fest installierten Träger 6, hier in Form einer einzigen, mittigen Säule 7, auf. Mittels einer geeigneten Einstelleinrichtung 8 ist der Träger 6 höhenverstellbar.

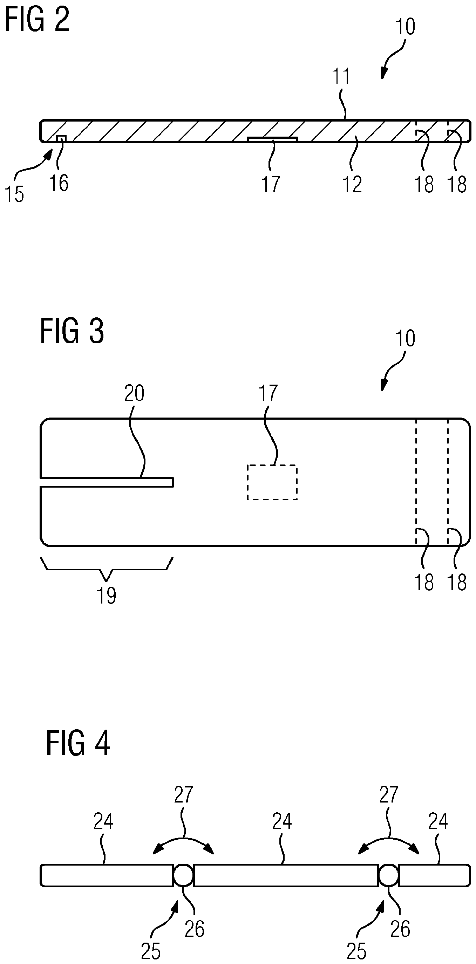

An dem Träger 6 ist über eine geeignete lösbare Befestigungseinrichtung 9 eine Patientenliege 10 befestigt gezeigt, die gemäß der Querschnittsdarstellung der Fig. 2 eine aus einem zweiten, flexiblen Material, hier Kunststoff, bestehende Hülle 11 aufweist, die ein erstes Material 12 umschließt, welches im vorliegenden Beispiel Natriumacetat-Trihydrat umfasst oder ist. Das erste Material vollzieht folglich vorliegend im erweiterten Raumtemperaturbereich einen Phasenübergang von einem festen, erstarrten, hier kristallinen Zustand in einen zähflüssigen, formbaren Zustand, weswegen die Patientenlagerungseinrichtung 1 auch eine in Fig. 1 angedeutete Heizeinrichtung 13 als Induzierungseinrichtung 14 aufweist. Eine zweite Induzierungseinrichtung 15 ist als Kristallisationsauslösevorrichtung 16, beispielsweise ein Piezoelement und/oder ein Knackplättchen, innerhalb der Hülle 11 aus dem zweiten Material, hier Kunststoff, vorgesehen. Die Nutzung der ersten Induzierungseinrichtung 14 ermöglicht es, die Patientenliege 10 über den Schmelzpunkt des ersten Materials 12 zur erwärmen, hier mithin über 58°C, so dass das erste Material 12 von dem festen Zustand in den formbaren Zustand übergeht. Das erste Material 12 bleibt vorliegend in einem metastabilen, formbaren, zähflüssigen Zustand, auch bei Abkühlung unter den Schmelzpunkt. Erst bei Betätigung der zweiten Induzierungseinrichtung 15 wird der Phasenübergang zurück in den festen Zustand induziert, wobei vorliegend eine gewisse Wärmeabgabe erfolgt, die durch eine Wärmeaufnahmeeinrichtung, insbesondere eine Kühleinrichtung, aufgefangen werden kann, um eine möglichst schnelle Erstarrung des ersten Materials 12 zu begünstigen.

Fig. 2 zeigt zudem angedeutete Befestigungsmittel 17 als Teil der lösbaren Befestigungseinrichtung 9, die beispielsweise eine in das zweite Material der Hülle 11 integrierte Platte mit Muttern aufweisen können.

Das zweite Material der Hülle 11 ist zudem auch elastisch ausgebildet, um zum einen die in dem ersten Material 12 bei den Phasenübergängen auftretende Volumenänderung kompensieren zu können, zum anderen wirkt die Elastizität des zweiten Materials der Hülle 11 auch darauf hin, eine möglichst gleiche Dicke über Ausdehnung der Patientenliege 10 aufrecht zu erhalten.

Hierzu können optional im Übrigen auch gestrichelt angedeutete, quer verlaufende Stege 18 mit Durchgangsöffnungen für das erste Material 12 verwendet werden, die die Form und somit die Dicke der Patientenliege 10 weiter stabilisieren, wie dies beispielsweise aus der Technik der Luftmatratzen bekannt ist.

Sowohl das erste Material 12 als auch das zweite Material weisen dabei vergleichbare Röntgenschwächungseigenschaften auf, wobei das zweite Material so gewählt ist, dass es im für die Phasenübergänge des ersten Materials 12 genutzten Zustandsgrößenbereich phasenstabil ist. Sowohl das erste Material 12 wie auch das zweite Material sind dabei röntgentransparent ausgebildet, das bedeutet, bei der vorliegenden Dicke der Patientenliege 10 liegt keine die Röntgenbildgebung mit der Röntgeneinrichtung 2 nennenswert beeinflussende Röntgenschwächung vor.

Es sei angemerkt, dass auch andere erste Materialien 12 herangezogen werden können, insbesondere auch Mischungen von Materialien, wie Schäume.

Wie die in Fig. 3 gezeigte Aufsicht auf die Patientenliege 10 zeigt, weist diese im für die Beine des Patienten vorgesehenen Beinbereich 19 einen in Längsrichtung verlaufenden Einschnitt 20 auf. Soll auch eine Aufspreizung der Beine in dem Sinne, dass wenigstens ein Bein seitlich (rechts/links) abgewinkelt gelagert ist, möglich sein, kann zusätzlich wenigstens ein in Querrichtung verlaufender Einschnitt vorhanden sein (in Fig. 3 nicht gezeigt).

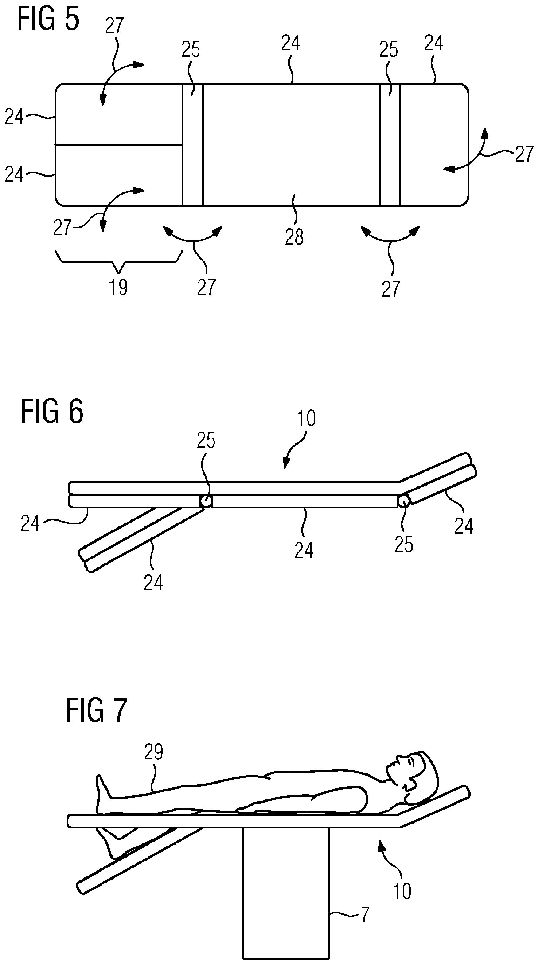

Die Verformbarkeit des ersten Materials 12 gemeinsam mit der Flexibilität der Hülle 11 im formbaren Zustand ermöglicht eine Anpassung der Patientenliege 10 auf den aktuell zu operierenden Patienten bzw. konkret die durchzuführende Operation, wobei, um die Anpassung zu vereinfachen, die Patientenlagerungseinrichtung 1 eine Vorbereitungseinrichtung 21 in Form eines Gestells 22 aufweist, die in Fig. 1 gezeigt ist. Der Auflagebereich 23 des Gestells 22 ist in den Figuren 4 und 5 in einer Seitenansicht bzw. einer Aufsicht gezeigt. Dabei weist der Auflagebereich vorliegend eine Mehrzahl von Teilplatten 24 auf, die über Verstelleinrichtungen 25, die ein Gelenk 26 umfassen, verstellbar verbunden sind, wie durch die Pfeile 27 angedeutet ist. Dabei sind vorliegend in Längsrichtung nur zwei Gelenkstellen gezeigt, was jedoch beispielhaft zu verstehen ist, da gerade die weitgehend formbare Ausgestaltung der Patientenliege 10 selbstverständlich auch deutlich mehr Verstellmöglichkeiten erlaubt. Ein Beispiel hierfür wäre auch die Anpassung an die Körpergröße. Beispielsweise kann die mittlere Teilplatte 24 dazu in ihrer Breite verstellbar sein, nachdem die flexible Hülle 11 ja beliebig abgewinkelt werden kann. Eine andere weitere Option wäre ein Spreizen der Beine.

Die Teilplatten 24 bilden in ihrer Gesamtheit eine Auflagefläche 28 für die Patientenliege 10. Wird die Patientenliege 10 mit dem ersten Material 12 im verformbaren Zustand auf die Auflagefläche 28 gelegt, kann sie in ihrer Form mittels der Verstelleinrichtungen 25 geeignet angepasst werden, wie dies in Fig. 6 gezeigt ist. Nachdem zwei Teilplatten 24 im Beinbereich 19 vorhanden sind, die unabhängig verkippt werden können, können für beide Beine des Patienten unterschiedliche Einstellungen gewählt werden, wie dies in Fig. 6 auch geschehen ist.

Nach der Formanpassung der Patientenliege 10 wird, vorliegend mittels der Induzierungseinrichtung 15, ein Phasenübergang zurück in den festen Zustand des ersten Materials 12 herbeigeführt. Ist dieser erst erreicht, kann, wie in Fig. 7 gezeigt, mittels der lösbaren Befestigungseinrichtung 9 die Patientenliege 10 wieder an der Säule 7 befestigt werden und, nachdem die Patientenliege 10 formstabil ist, der Patient 29 darauf platziert werden.

Wie bereits erwähnt wurde, sind im Rahmen der vorliegenden Erfindung auch andere erste Materialien und somit auch andere Arten von Induzierungseinrichtungen denkbar. Beispielsweise kann das erste Material einen Schaum umfassen. Der Schaum kann beispielsweise ein Trägermaterial für ein Latentwärmespeichermaterial sein, wobei der Schaum auf den Energiehaushalt des ersten Materials modifizierend wirken kann. Beispielsweise kann eine schnellere Wärmeabgabe, insbesondere an eine Wärmeabführungseinrichtung, begünstigt werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Patientenlagerungseinrichtung (1) für eine Röntgeneinrichtung (2) zum Einsatz bei einer Untersuchung eines Patienten (29), aufweisend eine Patientenliege (10) und wenigstens einen lösbar mit der Patientenliege verbundenen und die Patientenliege (10) tragenden Träger (6), wobei die Patientenliege (10) ein erstes, röntgentransparentes, in einer wenigstens teilweise flexiblen Hülle (11) aus einem zweiten, röntgentransparenten Material befindliches Material (12) aufweist, wobei das erste Material (12) einen festen, formstabilen Zustand und einen formbaren Zustand, insbesondere wenigstens teilweise flüssigen und/oder gasförmigen Zustand, aufweist, die durch reversible Phasenübergänge verknüpft sind, **dadurch gekennzeichnet, dass** die Patientenlagerungseinrichtung (1) ferner wenigstens eine Induzierungseinrichtung (14, 15) zur Induzierung wenigstens eines der Phasenübergänge des ersten Materials (12) zwischen den Zuständen durch einen Energieeintrag und/oder Energieentzug und zusätzlich zu dem Träger (6) eine Vorbereitungseinrichtung (21) für die Patientenliege aufweist, wobei die Vorbereitungseinrichtung (21) aufweist:
- wenigstens eine Auflagefläche(28) für die Patientenliege (10) und
- wenigstens eine auf die Auflagefläche (28) derart wirkende Verstelleinrichtung (25), dass durch Nutzung der Verstelleinrichtung (25) gezielt und definiert Verformungen der in einem formbaren Zustand aufgebrachten Patientenliege (10) herbeigeführt werden.

2. Patientenlagerungseinrichtung (1) nach Anspruch 1, **dadurch gekennzeichnet, dass** das zweite Material im für das erste Material (12) durch die Induzierungseinrichtung (14, 15) genutzten Zustandsbereich phasenstabil ist.

3. Patientenlagerungseinrichtung (1) nach Anspruch 1 oder 2, **dadurch gekennzeichnet, dass** das erste Material (12) im formbaren Zustand zähflüssig ist und/oder dass das erste Material (12) Wasser und/oder ein Latentwärmespeichermaterial, insbesondere Natriumacetat-Trihydrat, und/oder einen Schaum umfasst und/oder das zweite Material wenigstens einen Kunststoff umfasst oder ein Kunststoff ist.

4. Patientenlagerungseinrichtung (1) nach einem der vorangehenden Ansprüche, **dadurch gekennzeichnet, dass** das erste Material (12) und das zweite Material im Wesentlichen gleiche Schwächungseigenschaften für Röntgenstrahlung aufweisen.

5. Patientenlagerungseinrichtung (1) nach einem der vorangehenden Ansprüche, **dadurch gekennzeichnet, dass** das zweite Material zur Kompensation einer bei einem der Phasenübergänge auftretenden Volumenänderung und/oder zur Begünstigung einer Gleichverteilung einer Materialstärke des ersten Materials (12) elastisch ausgebildet ist und/oder wenigstens einen zur Formstabilisierung ausgebildeten, Durchtrittsöffnungen für das erste Material (12) aufweisenden Steg (18) aufweist und/oder nur an Sollgelenkstellen flexibel ist.

6. Patientenlagerungseinrichtung (1) nach einem der vorangehenden Ansprüche, **dadurch gekennzeichnet, dass** die Induzierungseinrichtung (14, 15) zur Erzeugung des Energieeintrags bzw. Energieentzugs durch Wärme und/oder Kälte und/oder elektrischen Strom ausgebildet ist.

7. Patientenlagerungseinrichtung (1) nach einem der vorangehenden Ansprüche, **dadurch gekennzeichnet, dass** wenigstens eine der wenigstens einen Verstelleinrichtung (25) ein Gelenk (26) zur Verkippung eines Anteils (24) der Auflagefläche (28) gegen einen benachbarten Anteil (24) der Auflagefläche (28) ist.

8. Patientenlagerungseinrichtung (1) nach einem der vorangehenden Ansprüche, **dadurch gekennzeichnet, dass** die Vorbereitungseinrichtung (21) ein Operationstisch oder ein Gestell (22) ist.

9. Patientenlagerungseinrichtung (1) nach einem der vorangehenden Ansprüche, **dadurch gekennzeichnet, dass** die Patientenliege (10) in dem Beinbereich (19) einen in Längsrichtung verlaufenden Einschnitt (20) zur unabhängigen Einstellung der Lagerfläche für beide Beine eines Patienten (29) in dem formbaren Zustand aufweist.

10. Patientenlagerungseinrichtung (1) nach einem der vorangehenden Ansprüche, **dadurch gekennzeichnet, dass** der insbesondere als wenigstens eine Säule (7) ausgebildete Träger (6) wenigstens in seiner Höhe verstellbar ist.

11. Verfahren zur Anpassung der Patientenliege (10) einer Patientenlagerungseinrichtung (1) nach einem der vorangehenden Ansprüche, umfassend die Schritte:
- Herstellen des formbaren Zustands des ersten Materials (12) durch Induzierung eines Phasenübergangs,
- Lösen der Patientenliege (10) vom Träger (6) und Platzieren der Patientenliege (10) auf der Auflagefläche (28) der Vorbereitungseinrichtung (21),
- Formanpassung der Patientenliege (10) mittels der Verstelleinrichtung (21),
- Herstellen des festen Zustands des ersten Materials (12) durch Induzierung eines Phasenübergangs, und
- Befestigung der Patientenliege (10) an dem Träger (6).

## Claims

1. Patient support device (1) for an x-ray device (2) for use during an examination of a patient (29), having a patient couch (10) and at least one support (6) bearing the patient couch (10) and connected detachably to the patient couch, wherein the patient couch (10) has a first, x-ray-transparent material (12) disposed in an at least partially flexible covering (11) made from a second x-ray-transparent material, wherein the first material (12) has a solid, dimensionally stable state and a deformable state, in particular at least partially liquid and/or gaseous state, which are linked by means of reversible phase transitions, **characterised in that** the patient support device (1) further has at least one induction device (14, 15) for inducing at least one of the phase transitions of the first material (12) between the states by means of applying and/or removing energy and in addition to the support (6) a preparation device (21) for the patient couch, wherein the preparation device (21) has:
- at least one contact surface (28) for the patient couch (10) and
- at least one adjusting device (25) acting on the contact surface (28) so that by using the adjusting device (25) deformations of the patient couch (10) attached in a deformable state can be effected in a targeted and defined manner.

2. Patient support device (1) according to claim 1, **characterised in that** the second material is phase stable in the state range used by the induction device (14, 15) for the first material (12).

3. Patient support device (1) according to claim 1 or 2, **characterised in that** the first material (12) in the deformable state is viscous and/or the first material (12) comprises water and/or a latent heat storage material, in particular sodium acetate trihydrate, and/or a foam and/or the second material comprises at least one plastic or is a plastic.

4. Patient support device (1) according to one of the preceding claims, **characterised in that** the first material (12) and the second material have essentially the same attenuation properties for x-ray radiation.

5. Patient support device (1) according to one of the preceding claims, **characterised in that** the second material is embodied elastically to compensate for a change in volume occurring in one of the phase transitions and/or for promoting a uniform distribution of a material strength of the first material (12), and/or has at least one web (18) embodied for form stabilisation and having through openings for the first material (12) and/or is only flexible at target hinge points.

6. Patient support device (1) according to one of the preceding claims, **characterised in that** the induction device (14, 15) is embodied to produce the application and/or removal of energy by means of heat and/or cold and/or electrical current.

7. Patient support device (1) according to one of the preceding claims, **characterised in that** at least one of the at least one adjusting device (25) is a hinge (26) for tilting a portion (24) of the contact surface (28) against an adjacent portion (24) of the contact surface (28).

8. Patient support device (1) according to one of the preceding claims, **characterised in that** the preparation device (21) is an operating table or a frame (22).

9. Patient support device (1) according to one of the preceding claims, **characterised in that** in the leg area (19) the patient couch (10) has a cut (20) running in the longitudinal direction for independently positioning the bearing surface for both legs of a patient (29) in the deformable state.

10. Patient support device (1) according to one of the preceding claims, **characterised in that** the support (6) embodied in particular as at least one pillar (7) can be adjusted at least in terms of its height.

11. Method for adjusting the patient couch (10) of a patient support device (1) according to one of the preceding claims, comprising the steps:
- producing the deformable state of the first material (12) by inducing a phase transition,
- detaching the patient couch (10) from the support (6) and positioning the patient couch (10) on the contact surface (28) of the preparation device (21),
- deforming the patient couch (10) by means of the adjusting device (21),
- producing the fixed state of the first material (12) by inducing a phase transition, and
- fastening the patient couch (10) to the support (6).

## Revendications

1. Dispositif (1) de support d'un patient d'un dispositif (2) à rayons X à utiliser lors d'un examen d'un patient (29) comportant une couchette (10) de patient et au moins un support (6) relié de manière amovible à la couchette du patient et portant la couchette (2) de patient, la couchette (10) du patient ayant un premier matériau (12) transparent aux rayons X et se trouvant dans une enveloppe (11) souple au moins en partie en un deuxième matériau transparent aux rayons X, le premier matériau (12) ayant un état solide de forme stable et un état déformable, notamment un état au moins en partie liquide et /ou gazeux, qui sont combinés par des transitions de phase réversibles, **caractérisé en ce que** le dispositif (1) de support d'un patient a en outre au moins un dispositif (14, 15) d'induction pour induire au moins l'une des transitions de phase du premier matériau (12) entre les états par un apport d'énergie et /ou un retrait d'énergie et en plus du support (6) un dispositif (21) de prétraitement de la couchette de patient, le dispositif (21) de prétraitement ayant :
- au moins une surface (28) de support de la couchette (10) du patient et
- au moins un dispositif (25) de déplacement agissant sur la surface (28) de support de manière à provoquer par l 'utilisation du dispositif (25) de déplacement des déformations ciblées et définies de la couchette (10) de patient déposée dans un état déformable.

2. Dispositif (20) de support de patient suivant la revendication 1, **caractérisé en ce que** le deuxième matériau est à phase stable dans le domaine d'état utilisé par le premier matériau (12) par le dispositif (14, 15) d'induction.

3. Dispositif (20) de support de patient suivant l'une des revendications 1 ou 2, **caractérisé en ce que** le premier matériau (2) est visqueux dans l'état déformable et /ou **en ce que** le premier matériau (12) comprend de l'eau et /ou un matériau à accumulation de chaleur latente, notamment de l 'acétate de sodium trihydraté et /ou une mousse et /ou **en ce que** le deuxième matériau comprend au moins une matière plastique ou est une matière plastique.

4. Dispositif (20) de support de patient suivant l'une des revendications précédentes, **caractérisé en ce que** le premier matériau (12) et le deuxième matériau ont sensiblement les mêmes propriétés d'atténuation des rayons X.

5. Dispositif (20) de support de patient suivant l'une des revendications précédentes, **caractérisé en ce que** le deuxième matériau est constitué élastiquement pour la compensation d 'une variation de volume se produisant lors de l'une des transitions de phase et /ou pour favoriser une répartition uniforme d'une épaisseur du premier matériau (12) et /ou a au moins une entretoise (18) constituée pour la stabilisation de forme et ayant des ouvertures de passage du premier matériau (12) et /ou n'est souple qu'en des points d'articulation de consigne.

6. Dispositif (20) de support de patient suivant l'une des revendications précédentes, **caractérisé en ce que** le dispositif (14, 15) d'induction est constitué pour la production de l'apport d'énergie ou du retrait d'énergie par de la chaleur et /ou du froid et /ou du courant électrique.

7. Dispositif (20) de support de patient suivant l'une des revendications précédentes, **caractérisé en ce qu'**au moins l 'un du au moins un dispositif (25) de déplacement est une articulation (26) pour le basculement d'une partie (24) de la surface (28) de support par rapport à une partie (24) voisine de la surface (28) de support.

8. Dispositif (20) de support de patient suivant l'une des revendications précédentes, **caractérisé en ce que** le dispositif (21) de prétraitement est une table d'opération ou un bâti (22).

9. Dispositif (20) de support de patient suivant l'une des revendications précédentes, **caractérisé en ce que** la couchette (10) de patient a dans l'état déformable, dans la partie (19) des jambes, une entaille (20) s'étendant dans la direction longitudinale pour régler indépendamment la surface de support des deux jambes d'un patient (29).

10. Dispositif (20) de support de patient suivant l'une des revendications précédentes, **caractérisé en ce que** le support (6) constitué notamment sous la forme d'au moins une colonne (7) est réglable au moins en hauteur.

11. Procédé d'adaptation de la couchette (10) de patient d 'un dispositif (20) de support de patient suivant l'une des revendications précédentes, comprenant les stades :
- on produit l'état déformable du premier matériau (12) par induction d'une transition de phase,
- on sépare la couchette (10) de patient du support (6) et on met la couchette (10) de patient sur la surface (28) de support du dispositif (21) de prétraitement,
- on adapte la forme de la couchette (10) de patient au moyen du dispositif (21) de réglage,
- on produit l'état solide du premier matériau (12) par induction d'une transition de phase et
- on fixe la couchette (10) de patient au support (6).
